# EUROPÄISCHE PATENTSCHRIFT

(11) **EP 1 280 939 B1**
(45) Veröffentlichungstag und Bekanntmachung des Hinweises auf die Patenterteilung: **07.05.2008**
(21) Anmeldenummer: 01936355.5
(22) Anmeldetag: 11.05.2001
(51) Int. Cl.: C12Q 1/68

(54) **VERFAHREN ZUM NACHWEIS VON POLYNUKLEOTIDEN**
METHOD FOR DETECTING POLYNUCLEOTIDES
PROCEDE DE DETECTION DE POLYNUCLEOTIDES

(30) Priorität: 12.05.2000 DE 10023421; 29.12.2000 DE 10065632
(43) Veröffentlichungstag der Anmeldung: 05.02.2003
(73) Patentinhaber: Gnothis Holding SA, 1015 Ecublens (CH)
(72) Erfinder: RIGLER, Rudolf, CH-1025 St-Sulpice (CH); FÖLDES-PAPP, Zeno, Clinical Immunology, 8036 Graz (AT)
(74) Vertreter: Weiss, Wolfgang
(86) Internationale Anmeldenummer: PCT/EP2001/005410
(87) Internationale Veröffentlichungsnummer: WO 2001/085991

(56) Entgegenhaltungen:
- EP-A- 0 679 251
- WO-A-01/00875
- WO-A-01/14589
- WO-A-97/14028
- US-A- 6 023 540
- RIGLER R ET AL: "Fluorescence cross-correlation: A new concept for polymerase chain reaction" JOURNAL OF BIOTECHNOLOGY, ELSEVIER SCIENCE PUBLISHERS B.V., AMSTERDAM, NL, Bd. 63, Nr. 2, 12. August 1998 (1998-08-12), Seiten 97-109, XP004148051 ISSN: 0168-1656 in der Anmeldung erwähnt
- AUER M. ET AL: 'FLUORESCENCE CORRELATION SPECTROSCOPY: LEAD DISCOVERY BY MINIATURIZED HTS' DRUG DISCOVERY TODAY Bd. 3, Nr. 10, 01 Oktober 1998, ELSEVIER SCIENCE LTD, GB, Seiten 457 - 465, XP000973192

## Beschreibung

Die Erfindung betrifft ein Verfahren zum Nachweis von Polynukleotiden auf einem Träger, wobei der Träger eine Vielzahl von separaten Nachweisbereichen mit jeweils unterschiedlichen Polynukleotiden enthält. Die Polynukleotide in den Nachweisbereichen liegen - im Gegensatz zu bekannten DNA-Chip-Testformaten - in einer nicht an den Träger gebundenen Form vor. Weiterhin wird eine Vorrichtung zur Durchführung des Verfahrens offenbart.

Die Verwendung sogenannter DNA-Chips zur Bestimmung von Polynukleotiden ist bekannt (siehe z.B. europäische Patente EP 0 373 203 und 0 619 321). DNA-Chips des Standes der Technik enthalten eine Vielzahl separater Nachweisbereiche auf der Oberfläche eines Trägers, die jeweils unterschiedliche Polynukleotide in einer trägergebundenen Form enthalten. Ein Nachteil dieser DNA-Chips besteht jedoch darin, dass sich die Reaktivität der auf dem Träger immobilisierten Polynukleotide signifikant von der Reaktivität freier Polynukleotide, wie sie in biologischen Systemen vorliegen, unterscheidet. Dies führt zu grundsätzlichen Problemen bei der Auswertung von Hybridisierungsexperimenten unter Verwendung gängiger DNA-Chips.

EP-A-0 679 251 offenbart ein Verfahren zur Detektion von Nukleinsäuren mittels Fluoreszenzkorrelationsspektroskopie.

Auer et al. (Drug Discovery Today, Band 3, Nr. 10, 1998, 457-465) beschreibt die Verwendung von Fluoreszenzkorrelationsspektroskopie für ein miniaturisiertes Hochdurchsatz-Screening.

US-A-6 023 540 offenbart einen faseroptischen Sensor unter Verwendung kodierter Partikel und dessen Anwendung in der Nukleinsäureanalytik.

WO 97/14028 offenbart ein Multiplexverfahren zur Diagnostik und genetischen Analytik, welches auf dem Einsatz von kodierten Partikeln beruht.

WO 01/14589 offenbart ein Nukleinsäuredetektionsverfahren auf der Basis farbkodierter Partikel.

Die der vorliegenden Erfindung zugrundeliegende Aufgabe bestand darin, neue Verfahren zur Bestimmung von Polynukleotiden bereitzustellen, die einerseits eine vergleichbare Effizienz wie gängige DNA-Chips aufweisen, bei denen aber andererseits die mit der Verwendung immobilisierter Polynukleotide auftretenden Nachteile beseitigt sind.

Diese Aufgabe wird gelöst durch ein Verfahren zur Bestimmung von Polynukleotiden, umfassend die Schritte:
(i) Bereitstellen eines Trägers, der mehrere separate Nachweisbereiche enthält, in denen sich jeweils ein Polynukleotid befindet, wobei das Polynukleotid in einer nicht an den Träger gebundenen Form vorliegt und an einen Mikropartikel gekoppelt ist, wobei die Größe des Mikropartikels von 10 nm bis ≤ 1 µm ist,
(ii) Inkontaktbringen der in den Nachweisbereichen befindlichen Polynukleotide mit Nachweissonden, wobei in einzelnen Nachweisbereichen jeweils verschiedene Kombinationen von Polynukleotid und Nachweissonde vorhanden sind, und
(iii) Bestimmen einer Hybridisierung der an den Mikropartikel gekoppelten Polynukleotide mit den Nachweissonden in Lösung durch konfokale Einzelmoleküldetektion, wobei die Bestimmung eine Untersuchung der Mobilität von Polynukleotid oder/und Nachweissonde umfasst.

Ein wesentlicher Vorteil des erfindungsgemäßen Verfahrens besteht darin, dass die Hybridisierung der zu untersuchenden Polynukleotide mit Nachweissonden in sehr geringen Messvolumina und bei sehr geringen Konzentrationen der Reaktionspartner durchgeführt werden kann. Hierzu wird vorzugsweise das im europäischen Patent 0 679 251 beschriebene Verfahren der Fluoreszenz-Korrelationsspektroskopie (FCS) oder ein anderes fluoreszenzspektroskopisches Verfahren mit ausreichender Sensitivität verwendet. Dies FCS umfasst vorzugsweise die Messung von einem oder wenigen Probenmolekülen in einem Messvolumen, wobei die Konzentration der zu bestimmenden Moleküle ≤ 10⁻⁶ mol/l beträgt und das Messvolumen vorzugsweise ≤ 10⁻⁴ I ist. Die Fluoreszenz-Korrelationsspektroskopie umfasst die Bestimmung stoffspezifischer Parameter, die durch Lumineszenzmessungen an den zu bestimmenden Molekülen ermittelt werden. Bei diesen Parametern kann es sich um Translationsdiffusionskoeffizienten, Rotationsdiffusionskoeffizienten oder/und um die Exzitationswellenlänge, die Emissionswellenlänge oder/und die Lebensdauer eines angeregten Zustandes eines lumineszierenden Substituenten oder die Kombination dieser Messgrößen handeln. Auf Einzelheiten zur Verfahrensdurchführung und apparative Details zu den für das Verfahren verwendeten Vorrichtungen wird auf die Offenbarung des europäischen Patents 0 679 251 verwiesen.

Alternativ kann auch eine Fluoreszenzabklingmessung durchgeführt werden, wobei man die Relaxationszeit der Fluoreszenzmarkierung bestimmt, oder das Auftreten von Energietransfer- oder Quenchvorgängen bestimmt werden.

Die in den Nachweisbereichen des Trägers angeordneten Polynukleotide sind vorzugsweise Nukleinsäuren wie DNA oder RNA, wobei DNA-Polynukleotide besonders bevorzugt sind. Andererseits können als Polynukleotide auch Nukleinsäureanaloga, wie etwa Peptidnukleinsäuren (PNA), eingesetzt werden. Die Polynukleotide können einerseits aus natürlichen Quellen stammen, z.B. aus Gen- oder cDNA-Bibliotheken, und Gene, cDNA-Moleküle oder Fragmente davon sein, andererseits kann es sich jedoch auch um synthetisch erzeugte, z.B. kombinatorische Polynukleotidsequenzen handeln. Die Polynukleotide liegen in einer Form vor, die unter den Bedingungen des Nachweisverfahrens mit einer komplementären Nachweissonde hybridisieren kann. Vorzugsweise liegen die Polynukleotide in einzelsträngiger Form vor.

Der Träger enthält bevorzugt mehrere Nachweisbereiche, in denen sich jeweils ein Polynukleotid mit unterschiedlicher Sequenz befindet. Es ist jedoch auch möglich, einen Träger mit mehreren Nachweisbereichen zu verwenden, die jeweils ein Polynukleotid mit gleicher Sequenz enthalten. In diesem Falle muss für jeden dieser Nachweisbereiche eine andere Nachweissonde eingesetzt werden. Die Länge der Polynukleotide in den Nachweisbereichen beträgt vorzugsweise mindestens 10 Nukleotide bis zu mehreren tausend Nukleotiden.

Die zur Untersuchung einer Hybridisierung mit den in den Nachweisbereichen befindlichen Polynukleotiden verwendeten Nachweissonden können - ebenso wie die Polynukleotide selbst - aus natürlichen oder synthetischen Quellen stammen. Die Nachweissonden tragen vorzugsweise eine oder mehrere Markierungsgruppen, diese Markierungsgruppen können während der Synthese der Nachweissonden (insbesondere bei chemischer Synthese oder bei Amplifikation) oder auch im Anschluss an die Synthese der Nachweissonden (z.B. durch enzymatische Anfügung am 5'- oder 3'-Ende) eingeführt werden. So können markierte Nachweissonden z.B. durch reverse Transkription von RNA-Molekülen, z.B. von mRNA-Molekülen unter Verwendung markierter Primer, oder durch enzymatische Primerextension unter Verwendung markierter Nukleotidbausteine erzeugt werden.

Erfindungsgemäß werden Polynukleotide verwendet, die an einen Mikropartikel gekoppelt sind. Dieser Mikropartikel hat eine Größe, die ausreicht, um die Diffusionsgeschwindigkeit eines daran gekoppelten Polynukleotids zu verlangsamen. Andererseits ist der Mikropartikel aber auch ausreichend klein genug, um sicherzustellen, dass das daran gekoppelte Polynukleotid in seinem Reaktionsverhalten im Wesentlichen einem "freien", nicht aber einen "immobilisierten" Polynukleotid entspricht. Die Größe der Mikropartikel ist 10 nm bis ≤ 1 µm, besonders bevorzugt 10 nm bis 500 nm. Bei den Mikropartikeln kann es sich um Latexpartikel z.B. aus Polystyrol oder anderen Polymeren, Metallsolpartikel, Silika-, Quarz- oder Glaspartikel handeln. Die Bindung von Polynukleotiden an Mikropartikel kann durch kovalente oder nicht-kovalente Wechselwirkungen erfolgen. Beispielsweise kann die Bindung der Polynukleotide an den Mikropartikel durch hochaffine Wechselwirkungen zwischen den Partnern eines spezifischen Bindepaares, z.B. Biotin/Streptavidin oder Avidin, Hapten/Anti-Hapten-Antikörper, Zucker/Lectin etc. vermittelt werden. So können biotinylierte Polynukleotide an Streptavidin-beschichtete Mikropartikel gekoppelt werden. Alternativ können auch die Polynukleotide adsorptiv an Partikel gebunden werden. So kann eine Bindung von durch Einbau von Alkanthiolgruppen modifizierten Polynukleotiden an Metallpartikel, z.B. Goldpartikel, erfolgen. Noch eine weitere Alternative ist die kovalente Immobilisierung, wobei die Bindung der Polynukleotide über reaktive Silangruppen auf einer Silikaoberfläche vermittelt werden kann. Gegebenenfalls können alternativ oder zusätzlich auch an Mikropartikel gekoppelte Nachweissonden eingesetzt werden.

Eine weitere Möglichkeit besteht darin, die Polynukleotide bzw. Nachweissonden durch trägergebundene Synthese am Partikel unter Verwendung bekannter Festphasensynthesemethoden herzustellen.

An einen Mikropartikel kann ein einziger Polynukleotidstrang gebunden werden. Es können jedoch auch mehrere, z.B. bis zu mehreren tausend Polynukleotidstränge an einen Partikel gebunden werden. Die Bindung erfolgt vorzugsweise über den 5'- oder den 3'-Terminus des Polynukleotidstrangs.

Für das erfindungsgemäße Verfahren wird ein Träger mit mehreren, vorzugsweise mit einer Vielzahl von 10² oder mehr separaten Nachweisbereichen verwendet. Die Nachweisbereiche sind so ausgestaltet, dass sie eine Hybridisierung des Polynukleotids mit der Nachweissonde in Lösung ermöglichen. Vorzugsweise handelt es sich bei den Nachweisbereichen um Vertiefungen in der Trägeroberfläche, wobei diese Vertiefungen grundsätzlich eine beliebige Form aufweisen können, z.B. kreisförmig, quadratisch, rautenförmig etc. Vorzugsweise enthalten die Nachweisbereiche ein Volumen von ≤ 10⁻⁶ I und besonders bevorzugt von ≤ 10⁻⁸ I. Der Träger enthält vorzugsweise 10³ oder mehr, besonders bevorzugt 10⁴ oder mehr separate Nachweisbereiche.

Bevorzugte Konzentrationen der zu bestimmenden Polynukleotide in den Nachweisbereichen sind ≤ 10⁻⁶ mol/l, besonders bevorzugt 10⁻¹⁰ bis 10⁻¹⁴ mol/l, während die Nachweissonden in einer Konzentration von vorzugsweise ≤ 10⁻⁴ mol/l, besonders bevorzugt 10⁻⁸ mol/l bis 10⁻¹¹ mol/l angeboten werden.

Für das erfindungsgemäße Verfahren werden vorzugsweise markierte Nachweissonden verwendet, wobei durch Lumineszenzmessungen nachweisbare Markierungen, insbesondere durch Fluoreszenzmessung nachweisbare Markierungen bevorzugt sind. Wenn die in einem Nachweisbereich verwendete Nachweissonde mit dem dort befindlichen Polynukleotid hybridisiert, findet aufgrund dieser Hybridisierung eine nachweisbare Änderung eines stoffspezifischen Parameters statt. Diese nachweisbare Änderung kann durch Fluoreszenzmessung gemessen werden. So erfolgt beispielsweise bei Hybridisierung von Nachweissonde und Polynukleotid eine nachweisbare Mobilitätsverringerung des Hybrids gegenüber den Ausgangskomponenten. Alternativ oder zusätzlich kann auch das Auftreten von Quench- oder Energietransfervorgängen bei der Hybridisierung nachgewiesen werden. Solche Energietransfervorgänge treten beispielsweise bei Verwendung mehrerer verschiedener markierter Sonden bzw. markierter Polynukleotide und markierter Sonden auf.

Die Detektion der an die Mikropartikel gekoppelten Polynukleotide erfolgt mittels konfokaler Einzelmoleküldetektion wie etwa durch Fluoreszenz-Korrelations-spektroskopie gemäß EP 0 679 251 erfolgen, bei der ein sehr kleines, vorzugsweise ein konfokales Volumenelement, beispielsweise 0,1 x 10⁻¹⁵ bis 20 x 10⁻¹² I dem Anregungslicht eines Lasers ausgesetzt wird, das die in diesem Messvolumen befindlichen fluoreszenzmarkierten Nachweissonden zur Emission von Fluoreszenzlicht anregt, wobei das emittierte Fluoreszenzlicht mittels eines Fotodetektors gemessen wird und eine Korrelation zwischen der zeitlichen Veränderung der gemessenen Emission und der Mobilität der Fluoreszenzmarkierungsgruppe bestimmt werden kann. Die konfokale Einzelmolekülbestimmung ist weiterhin bei Rigler und Mets (Soc.Photo-Opt.Instrum.Eng. 1921 (1993), 239 ff.) und Mets und Rigler (J.Fluoresc. 4 (1994), 259-264) beschrieben.

Alternativ bzw. zusätzlich kann die Detektion auch durch eine zeitaufgelöste Abklingmessung, ein sogenanntes Time Gating erfolgen, wie beispielsweise von Rigler et al: Picosecond Single Photon Fluorescence Spectroscopy of Nucleic Acids, in: "Ultrafast Phenomena", D.H. Auston ed. Springer 1984, beschrieben. Dabei erfolgt die Anregung der Fluoreszenzmoleküle innerhalb eines Messvolumens und anschließend - vorzugsweise in einem zeitlichen Abstand von ≥ 100 ps - das Öffnen eines Detektionsintervalls am Fotodetektor. Auf diese Weise können durch Ramaneffekte erzeugte Hintergrundsignale ausreichend gering gehalten werden, um eine im Wesentlichen störungsfreie Detektion zu ermöglichen. Das Timegating ist besonders zur Messung von Quench- oder Energietransfervorgängen geeignet.

In einer bevorzugten Ausführungsform des Verfahrens kann die Bestimmung auch die Messung eines kreuzkorrelierten Signals umfassen, das von einem mindestens 2 unterschiedliche Markierungen, insbesondere Fluoreszenzmarkierungen, enthaltenden Sonden-Polynukleotidkomplex stammt, wobei mehrere unterschiedlich markierte Sonden oder/und Polynukleotide mit jeweils unterschiedlichen Markierungen eingesetzt werden können. Diese Kreuzkorrelationsbestimmung ist beispielsweise bei Schwille et. al. (Biophys.J. 72 (1997), 1878-1886) und Rigler et. al. (J.Biotechnol. 63 (1998), 97-109) beschrieben.

Der für das Verfahren verwendete Träger sollte so ausgestaltet sein, dass er eine optische Detektion in den Nachweisbereichen ermöglicht. Vorzugsweise wird daher ein zumindest in den Nachweisbereichen optisch transparenter Träger verwendet. Der Träger kann dabei entweder vollständig optisch transparent sein oder eine optisch transparente Basis und eine optisch undurchlässige Deckschicht mit Aussparungen in den Nachweisbereichen enthalten. Geeignete Materialien für Träger sind beispielsweise Verbundstoffträger aus Metall (z.B. Silicium für die Deckschicht) und Glas (für die Basis). Derartige Träger können beispielsweise Verbundstoffträger aus Metall (z.B. Silicium für die Deckschicht) und Glas (für die Basis). Derartige Träger können beispielsweise durch Aufbringen einer Metallschicht mit vorgegebenen Aussparungen für die Nachweisbereiche auf das Glas erzeugt werden. Alternativ können Plastikträger, z.B. aus Polystyrol oder Polymeren auf Acrylat- oder Methacrylatbasis eingesetzt werden.

Ein weiterer Gegenstand der Erfindung betrifft die Verwendung einer Vorrichtung nach einem der Ansprüche 1-12, wobei die Vorrichtung umfasst einen Träger, der mehrere separate Nachweisbereiche enthält, in denen sich jeweils ein Polynukleotid befindet, wobei das Polynukleotid in einer nicht an den Träger gebundenen Form vorliegt. Die Vorrichtung kann durch Aufbringen der Polynukleotide in die Nachweisbereiche des Trägers in Form einer Lösung, vorzugsweise einer wässrigen Lösung, und anschließendes Eintrocknen hergestellt werden. Die auf diese Weise erzeugte Vorrichtung ist für lange Zeit, z.B. mehrere Monate, stabil.

Noch ein weiterer Gegenstand der Erfindung ist die Verwendung eines Reagenzienkits nach einem der Ansprüche 1-12 zur Bestimmung von Polynukleotiden, wobei der Reagenzienkit umfasst eine Vorrichtung wie vorausgehend beschrieben und (a) einen Satz markierter Nachweissonden oder (b) Mittel zur Herstellung eines Satzes markierter Nachweissonden, z.B. markierte Primer oder markierte (Deoxy)ribonukleosidtriphosphate, sowie gegebenenfalls Enzyme zur Herstellung geeigneter Nachweissonden.

Das erfindungsgemäße Verfahren kann beispielsweise für die funktionelle Genomik und zur Transkriptomanalyse eingesetzt werden.

Weiterhin soll die vorliegende Erfindung durch die nachfolgenden Figuren und Beispiele erläutert werden. Es zeigen:

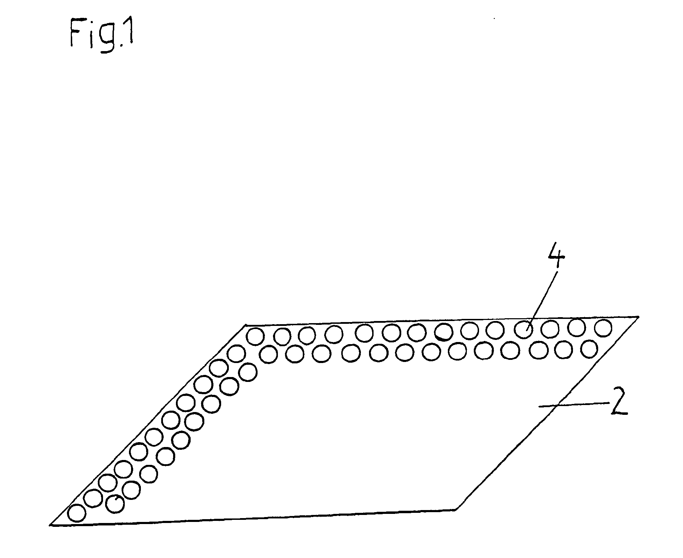

Figur 1: die schematische Darstellung eines zur Durchführung des erfindungsgemäßen Verfahrens geeigneten Trägers (2) mit einer Vielzahl von in Form von Vertiefungen auf dem Träger ausgebildeten Nachweisbereichen (4). Ein Träger mit einer Fläche von 1 bis 2 cm² kann beispielsweise bis zu 10⁴ Vertiefungen enthalten.

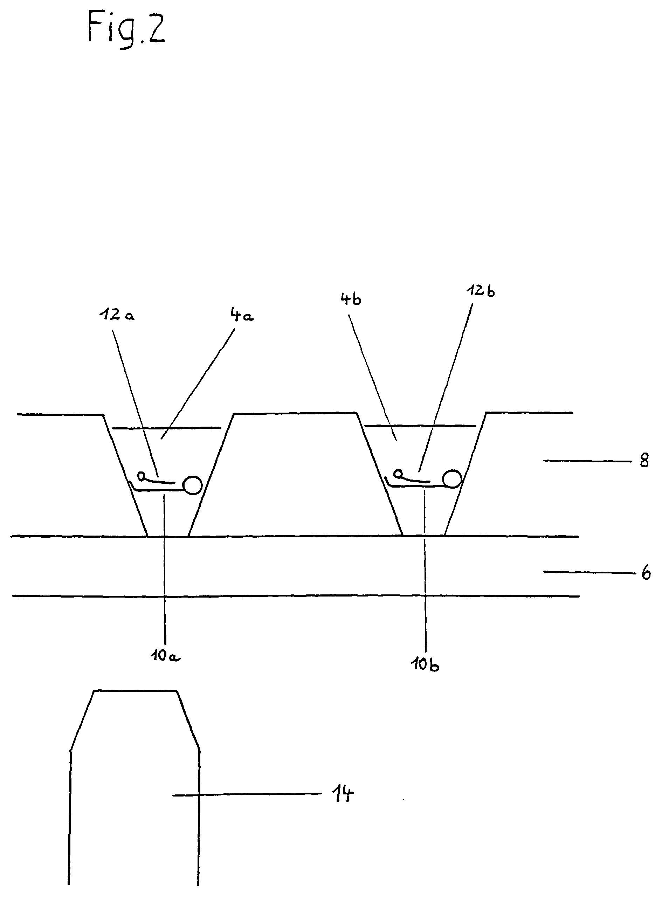

Figur 2: einen Querschnitt durch einen erfindungsgemäßen Träger. Der Träger enthält eine Basis (6), vorzugsweise aus einem optisch transparenten Material wie Glas, und eine Deckschicht (8) mit Aussparungen, z.B. aus Silicium. Es sind zwei Nachweisbereiche (4a, 4b) gezeigt, die jeweils ein an einen Mikropartikel gekoppeltes Polynukleotid (10a, 10b) und eine Nachweissonde (12a, 12b) enthalten. Im Nachweisbereich (4a) findet eine Hybridisierung des Polynukleotids (10a) mit der Nachweissonde (12a) statt, während im Nachweisbereich (4b) keine Hybridisierung zwischen dem Polynukleotid (10b) und der Nachweissonde (12b) erfolgt. Der Nachweis einer Hybridisierung zwischen Mikropartikel-gekoppeltem Polynukleotid (10a) und markierter Nachweissonde (12a) kann durch Untersuchung von Einzelmolekülen erfolgen.

Für den Nachweis kann eine vorzugsweise unter der Trägerbasis angeordnete Detektionsvorrichtung (14) eingesetzt werden. Die Detektionsvorrichtung (14) kann einen Laser und einen Detektor enthalten. Vorzugsweise wird eine Laser-Detektormatrix bestehend aus einer Punktmatrix von Laserpunkten erzeugt durch eine Diffraktionsoptik oder einen Quanten-Well-Laser sowie einer Detektormatrix erzeugt durch fasergekoppelte einzelne Avalanche-Fotodiodendetektoren oder eine Avalanche-Fotodiodenmatrix verwendet. Alternativ kann auch eine elektronische Detektormatrix, z.B. eine CCD-Kamera eingesetzt werden.

### Beispiel

In einer Matrix von m x n Mikrovertiefungen auf einem oder mehreren Träger(n) wird eine Bibliothek von 4ⁿ unterschiedlichen Nukleotidsequenzen der Länge m erzeugt, wobei jede Sequenz in einen separaten Nachweisbereich transferiert wird. Die Nukleotidsequenzen enthalten an ihrem 5'-Ende eine Biotingruppe und werden an Streptavidin-beschichtete Mikropartikel gekoppelt.

Eine mRNA- oder cDNA-Bibliothek wird durch enzymatische Reaktion, z.B. mit terminaler Transferase oder Ligase am 3'- und/oder 5'-Ende mit fluoreszenzmarkierten Nukleotiden versehen. Die markierten Nukleotide werden in die Vertiefungen des Trägers pipettiert.

Eine spezifische Hybridisierung zwischen Polynukleotid und markierter Nachweissonde erfolgt durch Analyse der Translationsbewegung unter Verwendung konfokaler Einzelmolekülanalyse. Der Nachweis erfolgt durch eine Laser-Detektormatrix.

## Patentansprüche

1. Verfahren zur Bestimmung von Polynukleotiden, umfassend die Schritte:
(i) Bereitstellen eines Trägers, der mehrere separate Nachweisbereiche enthält, in denen sich jeweils ein Polynukleotid befindet, wobei das Polynukleotid in einer nicht an den Träger gebundenen Form vorliegt und an einen Mikropartikel gekoppelt ist, wobei die Größe des Mikropartikels von 10 nm bis s 1 µm ist,
(ii) Inkontaktbringen der in den Nachweisbereichen befindlichen Polynukleotide mit Nachweissonden, wobei in einzelnen Nachweisbereichen jeweils verschiedene Kombinationen von Polynukleotid und Nachweissonde vorhanden sind, und
(iii) Bestimmen einer Hybridisierung der an Mikropartikel gekoppelten Polynukleotide mit den Nachweissonden in Lösung durch konfokale Einzelmoleküldetektion, wobei die Bestimmung eine Untersuchung der Mobilität von Polynukleotid oder/und Nachweissonde umfasst.

2. Verfahren nach Anspruch 1,
**dadurch gekennzeichnet,**
**dass** die Konzentration der Polynukleotide in den Nachweisbereichen ≤10⁻⁶ mol/l beträgt.

3. Verfahren nach einem der Ansprüche 1 oder 2,
**dadurch gekennzeichnet,**
**dass** die Nachweisbereiche ein Volumen von jeweils ≤10⁻⁶ I oder weniger aufweisen.

4. Verfahren nach einem der Ansprüche 1 bis 3,
**dadurch gekennzeichnet,**
**dass** der Träger 10³ oder mehr separate Nachweisbereiche enthält.

5. Verfahren nach einem der Ansprüche 1 bis 4,
**dadurch gekennzeichnet,**
**dass** man eine markierte Nachweissonde verwendet.

6. Verfahren nach einem der Ansprüche 1 bis 5,
**dadurch gekennzeichnet,**
**dass** man eine fluoreszenzmarkierte Nachweissonde verwendet.

7. Verfahren nach einem der Ansprüche 1 bis 6,
**dadurch gekennzeichnet,**
**dass** die Bestimmung durch Fluoreszenzkorrelationsspektroskopie oder/und durch zeitaufgelöste Abklingmessung erfolgt.

8. Verfahren nach einem der Ansprüche 1 bis 7,
**dadurch gekennzeichnet,**
**dass** die Bestimmung eine Untersuchung des Auftretens von Quench- oder Energietransfervorgängen umfasst.

9. Verfahren nach einem der Ansprüche 1 bis 8,
**dadurch gekennzeichnet,**
**dass** die Bestimmung die Messung eines kreuzkorrelierten Signals umfasst, das von einem, mindestens 2 unterschiedliche Markierungen, insbesondere Fluoreszenzmarkierungen, enthaltenden Sonden-Polynukleotidkomplex stammt.

10. Verfahren nach einem der Ansprüche 1 bis 9,
**dadurch gekennzeichnet,**
**dass** man einen zumindest in den Nachweisbereichen optisch transparenten Träger verwendet.

11. Verfahren nach einem der Ansprüche 1 bis 10,
**dadurch gekennzeichnet,**
**dass** der Träger eine optisch transparente Basis und eine optisch undurchlässige Deckschicht mit Aussparungen in den Nachweisbereichen enthält.

12. Verfahren nach einem der Ansprüche 1 bis 11,
**dadurch gekennzeichnet,**
**dass** man einen Metall/Glas-Träger oder einen Plastikträger verwendet.

13. Verwendung einer Vorrichtung in einem Verfahren nach einem der Ansprüche 1 bis 12, wobei die Vorrichtung umfasst:
einen Träger, der mehrere separate Nachweisbereiche enthält, in denen sich jeweils ein Polynukleotid, das an einen Mikropartikel gekoppelt ist, befindet, wobei das Polynukleotid in einer nicht an den Träger gebundenen Form vorliegt.

14. Verwendung eines Reagenzienkits in einem Verfahren nach einem der Ansprüche 1 bis 12, wobei der Reagenzienkit umfasst:
eine Vorrichtung umfassend einen Träger, der mehrere separate Nachweisbereiche enthält, in denen sich jeweils ein Polynukleotid, das an einen Mikropartikel gekoppelt ist, befindet, wobei das Polynukleotid in einer nicht an den Träger gebundenen Form vorliegt und (a) einen Satz markierter Nachweissonden oder (b) Mittel zur Herstellung eines Satzes markierter Nachweissonden.

## Claims

1. A method for determining polynucleotides, comprising the steps:
(i) providing a support containing two or more separate detection zones in which a polynucleotide is in each case located, the polynucleotide being present in a form which is not bound to the support and being coupled to a microparticle, the size of the microparticle being from 10 nm to ≤ 1 µm,
(ii) bringing the polynucleotides located in the detection zones into contact with detection probes, different combinations of polynucleotide and detection probe in each case being present in the individual detection zones, and
(iii) determining hybridisation of the microparticle-coupled polynucleotides with the detection probes in solution by confocal single molecule detection, said determination comprising an investigation of the mobility of the polynucleotide and/or detection probe.

2. A method according to claim 1,
**characterised in that**
the concentration of the polynucleotides in the detection zones amounts to ≤ 10⁻⁶ mol/l.

3. A method according to either of claim 1 or claim 2,
**characterised in that**
the detection zones have a volume of in each case ≤ 10⁻⁶ 1 or less.

4. A method according to any one of claims 1 to 3,
**characterised in that**
the support contains 10³ or more separate detection zones.

5. A method according to any one of claims 1 to 4,
**characterised in that**
a labelled detection probe is used.

6. A method according to any one of claims 1 to 5,
**characterised in that**
a fluorescently labelled detection probe is used.

7. A method according to any one of claims 1 to 6,
**characterised in that**
determination proceeds by fluorescence correlation spectroscopy and/or by time-resolved decay measurement.

8. A method according to any one of claims 1 to 7,
**characterised in that**
determination involves an investigation of the occurrence of quenching or energy transfer processes.

9. A method according to any one of claims 1 to 8,
**characterised in that**
determination comprises a measurement of a cross-correlated signal originating from a probe/polynucleotide complex containing at least 2 different labels, in particular fluorescence labels.

10. A method according to any one of claims 1 to 9,
**characterised in that**,
at least in the detection zones, an optically transparent support is used.

11. A method according to any one of claims 1 to 10,
**characterised in that**
the support comprises an optically transparent base and an optically opaque outer layer with recesses in the detection zones.

12. A method according to any one of claims 1 to 11,
**characterised in that**
a metal/glass support or a plastics support is used.

13. Use of a device in a method according to any one of claims 1 to 12, the device comprising:
a support containing two or more separate detection zones, in which there is located in each case a polynucleotide, which is coupled to a microparticle, the polynucleotide being present in a form which is not bound to the support.

14. Use of a reagent kit in a method according to any one of claims 1 to 12, the reagent kit comprising:
a device comprising a support containing two or more separate detection zones, in which there is located in each case a polynucleotide, which is coupled to a microparticle, the polynucleotide being present in a form which is not bound to the support and (a) a set of labelled detection probes or (b) means for producing a set of labelled detection probes.

## Revendications

1. Procédé pour la détermination de polynucléotides, comprenant les étapes consistant à :
(i) mettre à disposition un support qui comprend plusieurs zones de détection séparées, dans chacune desquelles se trouve un polynucléotide, le polynucléotide se trouvant dans une forme non liée au support et étant couplé à une microparticule, la dimension de la microparticule étant de 10 nm à ≤ 1 µm.,
(ii) mettre en contact les polynucléotides se trouvant dans les zones de détection avec les sondes de détection, dans chacune des zones de détection, se trouvant des combinaisons différentes de polynucléotides et de sondes de détection, et
(iii) déterminer une hybridation des polynucléotides couplés aux microparticules avec les sondes de détection dans la solution par détection moléculaire individuelle à foyer commun, la détermination comprenant une analyse de la mobilité du polynucléotide et/ou de la sonde de détection.

2. Procédé selon la revendication 1,
**caractérisé en ce que**
la concentration des polynucléotides dans les zones de détection est de ≤10⁻⁶ mole/l.

3. Procédé selon l'une des revendications 1 ou 2,
**caractérisé en ce que**
les zones de détection présentent chacun un volume de ≤10⁻⁶ l ou moins.

4. Procédé selon l'une des revendications 1 à 3,
**caractérisé en ce que**
le support contient 10³ ou davantage de zones de détection séparées.

5. Procédé selon l'une des revendications 1 à 4,
**caractérisé en ce que**
l'on utilise une sonde de détection marquée.

6. Procédé selon l'une des revendications 1 à 5,
**caractérisé en ce que**
l'on utilise une sonde de détection marquée par fluorescence.

7. Procédé selon l'une des revendications 1 à 6,
**caractérisé en ce que**
la détermination s'effectue par spectroscopie de corrélation par fluorescence ou/et par mesure de la décroissance résolue dans le temps.

8. Procédé selon l'une des revendications 1 à 7,
**caractérisé en ce que**
la détermination comprend une analyse de la survenue de processus d'extinction ou de transfert d'énergie.

9. Procédé selon l'une des revendications 1 à 8,
**caractérisé en ce que**
la détermination comprend la mesure d'un signal à corrélation croisée qui provient d'un complexe polynucléotide-sonde, contenant au moins deux marquages différents, en particulier des marquages par fluorescence.

10. Procédé selon l'une des revendications 1 à 9,
**caractérisé en ce que**
l'on utilise un support optiquement transparent au moins dans les zones de détection.

11. Procédé selon l'une des revendications 1 à 10,
**caractérisé en ce que**
le support contient une base optiquement transparente et une couche supérieure optiquement non transparente avec des évidements dans les zones de détection.

12. Procédé selon l'une des revendications 1 à 11,
**caractérisé en ce que**
l'on utilise un support métal/verre ou un support plastique.

13. Utilisation d'un dispositif dans un procédé selon l'une des revendications 1 à 12, dans laquelle le dispositif comprend :
un support qui contient plusieurs zones de détection séparées, dans chacune desquelles se trouve un polynucléotide couplé à une microparticule, le polynucléotide se présentant sous une forme non liée au support.

14. Utilisation d'un kit d'agents réactifs dans un procédé selon l'une des revendications 1 à 12, dans laquelle le kit d'agents réactifs comprend :
un dispositif comprenant un support qui contient plusieurs zones de détection séparées, dans chacune desquelles se trouve un polynucléotide couplé à une microparticule, le polynucléotide se trouvant sous une forme non liée au support, et (a) un jeu de sondes marquées ou (b) des moyens pour la préparation d'un jeu de sondes de détection marquées.
